# EUROPÄISCHE PATENTSCHRIFT

(11) **EP 0 335 884 B1**
(45) Veröffentlichungstag und Bekanntmachung des Hinweises auf die Patenterteilung: **05.05.1993**
(21) Anmeldenummer: 87907935.8
(22) Anmeldetag: 08.12.1987
(51) Int. Cl.: C12Q 1/28, G01N 33/535

(54) **IMMUNENZYMATISCHER TEST MIT DETEKTION DURCH FARBUMSCHLAG IM SICHTBAREN SPEKTRALBEREICH**
IMMUNOLOGICAL ENZYMATIC TEST WITH DETECTION BY COLOR CHANGES IN THE VISIBLE SPECTRUM
TEST IMMUNOLOGIQUE A ENZYMES DE DEPISTAGE PAR CHANGEMENT DE COULEURS DANS LE SPECTRE VISIBLE

(30) Priorität: 08.12.1986 DE 3641830
(43) Veröffentlichungstag der Anmeldung: 11.10.1989
(73) Patentinhaber: FRAUNHOFER-GESELLSCHAFT ZUR FÖRDERUNG DER ANGEWANDTEN FORSCHUNG E.V., 80636 München (DE)
(72) Erfinder: Bryniok, Dieter, D-7409 Dusslingen (DE); Trick, Iris, D-7540 Neuenburg (DE); Trösch, Walter, D-7000 Stuttgart 61 (DE)
(74) Vertreter: Patentanwälte Ruff, Beier, Schöndorf und Mütschele
(86) Internationale Anmeldenummer: DE8700583
(87) Internationale Veröffentlichungsnummer: WO8804328

(56) Entgegenhaltungen:
- EP-A- 0 224 210
- WO-A-85/05688
- WO-A-86/05207
- FR-A- 2 449 882
- US-A- 4 503 143
- Clinical Chemistry, vol. 28, No. 4, April 1982, (Washington, US) R.H. White-Stevens: "Interference by asorbic acid in test systems involving peroxidase. I. Reversible indicators and the effects of copper, iron, and mercury", pp. 578-588, s. p. 579, column 2, lines 20-38

## Beschreibung

Die Erfindung bezieht sich auf ein Verfahren zur immunenzymatischen Bestimmung von Antigenen oder Antikörpern mit Detektion durch Farbumschlag in sichtbarem Spektralbereich.

Die enzymatische Analyse ist vor allem dank der Spezifität und Geschwindigkeit eine gegenüber den herkömmlichen überlegene Analysenmethode. Eine der wichtigsten Varianten der enzymatischen Analyse für die Bestimmung von Antigenen oder Antikörpern ist der ELISA (enzyme-linked immunosorbent assay). Der ELISA in seiner einfachsten Form dient zum Nachweis einer beliebigen Substanz (Antigen), gegen die Antikörper verfügbar sind. Das nachzuweisende Antigen wird dabei an eine feste Oberfläche adsorbiert. Danach wird das fixierte Antigen mit Antikörpern markiert, die zuvor kovalent an ein Indikatorenzym gebunden wurden. Nach dem Auswaschen der überschüssigen Antikörper wird das Enzym durch eine Farbreaktion nachgewiesen. Durch die Konzentration des gebildeten farbigen Reaktionsproduktes kann auf die Konzentration des Antigens zurückgeschlossen werden. In analoger Weise ist die Bestimmung von Antikörpern mit einem zweiten Antikörper möglich.

Es ist bekannt, daß diese "colorometrischen ELISAs" vor allem mit drei Enzymen durchgeführt werden: alkalische Phosphatase mit p-Nitrophenylphosphat als Substrat, β-Galactosidase mit o-Phenyl-β-D-Galactopyranosid und Peroxidase mit verschiedenen Farbsubstraten. Die quantitative Messung erfolgt bei allen ELISAs durch Bestimmung der Konzentration des farbigen Reaktionsproduktes, das aus dem Enzym und dem zuerst farblosen Substrat gebildet wird.

Bei diesen bekannten Methoden wird jedoch ein hoher Substratüberschuß verwendet, der in der Regel um den Faktor 10 über dem des tatsächlichen Substratumsatzes liegt. Dadurch soll erreicht werden, daß das Enzym mit max. Umsatzgeschwindigkeit arbeitet, was die Empfindlichkeit und Schnelligkeit des Testes anheben soll. Durch diese Maßnahme wird jedoch ein farbiges Reaktionsprodukt gebildet, dessen quantitative Bestimmung nur durch eine aufwendige und zeitraubende photometrische Analyse erfolgen kann, die zudem von ausgebildetem Personal ausgeführt werden muß. Eine einfache, billige und schnelle Analyse ohne Laborausrüstung ist demnach nicht möglich.

ELISA-Verfahren, die Peroxidase als Enzym und Tetramethylbenzidin (TMB) als Farbsubstrat verwenden, sind in der Literatur beschrieben. Aus der Druckschrift von H. Gallati und I. Pracht (J. Clin. Chem. Clin. Biochem. 23, 453-460) ist z. B. bekannt, daß für ELISAs, für die 3,3',5,5'-Tetramethylbenzidin (TMB) als Substrat für Peroxidase verwendet wird, 1 µmol/ml TMB eingesetzt wird.

Eine ähnliche Bestimmungsmethode wird von E. Bos und A. van der Doelen et al. (J. Immunoassay 2, 187-204) beschrieben. Hier werden 420 nmol/ml TMB verwendet.

Im US-Patent 4 503 143 werden Peroxidase und TMB verwendet, wobei die Endkonzentration von TMB 43 mg/l, entsprechend 180 nmol/ml beträgt.

Beim Verfahren nach der Internationalen Anmeldung WO 86/05207 werden ebenfalls Peroxidase und TMB verwendet. Hier beträgt die Konzentration von TMB 0,01 - 0,1 mg/ml, entsprechend 42 - 420 nmol/ml.

In allen diesen Fällen wird das stabile blaugefärbte 3,3',5,5'-Tetramethylbenzidin-Radikalkation erhalten, der Farbumschlag zum gelben 3,3',5,5'-Tetramethyl-1,1'-Diphenochinon-4,4'-Diimmoniumion wird bedingt durch den vorhandenen großen Substratüberschuß nicht erreicht. Es wird somit nur ein farbiges Reaktionsprodukt angehäuft, d. h. es liegen keine Mischfarben vor. Die Unterschiede in der Helligkeit im blauen Bereich des Farbstoffs lassen eine gewisse qualitative Beurteilung zu. Eine quantitative Bestimmung ist jedoch nur mit Hilfe eines Spektralphotometers möglich.

Analoges gilt für die Bestimmungsmethode, bei der durch Abstoppung mit Säure dann nur das gelbe Reaktionsprodukt erhalten wird. Auch hier erfolgt die quantitative Auswertung durch eine aufwendige photometrische Analyse.

Ein solches Verfahren ist in Clin. Chem. 28 (1982), S. 578 - 588 beschrieben, bei dem Glucose-Oxidase und Peroxidase als Enzyme und o-Tolidin als Farbsubstrat in einem Glucose-Nachweistest verwendet werden. Die bei der Enzymreaktion gebildete blaue Farbe des Indikators wird durch Säurezugabe vollständig in die gelb gefärbte Form überführt und diese dann durch ein Photometer analysiert. Die Farbveränderung wird hierbei nach der Enzymreaktion vorgenommen, so daß keine Mischfarbe erzeugt und ausgewertet wird.

Es ist daher Aufgabe der Erfindung, eine quantitative, einfache, billige und schnelle immunenzymatische Bestimmungsmethode zur Verfügung zu stellen. Die Analysenmethode soll weiterhin von einer Laborausrüstung unabhängig sein.

Die Aufgabe wird gelöst durch ein Verfahren zur immunenzymatischen Bestimmung von Antigenen mit einem ersten Antikörper oder von Antikörpern mit einem zweiten Antikörper und einem an den ersten bzw. zweiten Antikörper kovalent gebundenen Indikatorenzym mittels eines Farbstoffes, indem eine Farbreaktion mit dem Indikatorenzym durchgeführt wird, das dadurch gekennzeichnet ist, daß die Farbstoffkonzentration nur in der Größenordnung des Substratumsatzes liegt, so daß in Abhängigkeit der Antigen- bzw. Antikörperkonzentration unterschiedlich gefärbte Reaktionsprodukte entstehen, und daß die quantitative Bestimmung des Antigens bzw. Antikörpers durch einen visuellen Vergleich der farbigen Reaktionsprodukte mit Referenzfarben mit dem menschlichen Auge erfolgt.

Die erfindungsgemäße Lösung zeigt, daß mit der Senkung der Farbstoffkonzentration in die Größenordnung des Substratumsatzes, die je nach Antigen- bzw. Antikörperumsatz unterschiedlich gefärbten Reaktionsprodukte zur einfachen und schnellen sowie genauen quantitativen Bestimmung dienen können.

Anhand des ELISA, für den TMB als Substrat für Peroxidasen verwendet wird, soll die Erfindung näher erläutert werden.

TMB wird dabei als Peroxidasesubstrat zunächst zum Zwischenprodukt, dem Tetramethylbenzidin-Radikalkation oxidiert, das mit einem Ladungsaustauschkomplex im Gleichgewicht steht. Dieses Zwischenprodukt ist blau und hat zwei Absorptionsmaxima bei 370 nm und 650 nm. Wird mehr H₂O₂ umgesetzt als der halben molaren Konzentration TMB entspricht, wird das Radikalkation weiter oxidiert zum Endprodukt dem 3,3',5,5'-Tetramethyl-1,1'-diphenochinon-4,4'-diimmoniumion, das bei einem Absorptionsmaximum von 450 nm gelb ist. Optisch vollzieht sich dieser Teil der Reaktion als ein kontinuierlicher Wechsel der Farbe der Lösung von blau über grüne Zwischentöne nach gelb. Die Erfindung ermöglicht, daß das auf Farbunterschiede sehr empfindlich reagierende menschliche Auge, die je nach Antigen- bzw. Antikörperumsatz verschiedenen farbigen Reaktionsprodukte durch Vergleich mit Referenzfarben schnell und quantitativ auswerten kann.

Im herkömmlichen ELISA, der mit Peroxidase und TMB als Substrat durchgeführt wird, werden an der oberen Grenze des Meßbereichs ca. 50 nmol H₂O₂/ml (äquivalent zum Substratumsatz) umgesetzt; d. h. der Meßbereich des ELISAs im Photometer umfaßt den Umsatz von 0 - 50 nmol H₂O₂/ml. Bei einer Auswertung des Tests kann eine weitere Akkumulation des blauen Zwischenprodukts bis ca. 100 nmol/ml erkannt werden. Wird der Farbumschlag zur Detektion ausgenützt, kann durch die Wahl der Farbstoffkonzentration der Meßbereich stark variiert werden. Die obere Grenze des Meßbereichs wird durch die TMB-Konzentration festgelegt. Wird der Test mit Glucose-Oxidase und Peroxidase im gekoppelten Enzymsystem durchgeführt, liegt die maximale Obergrenze bedingt durch die Löslichkeit des Sauerstoffs bei 250 nmol/ml. Wird die TMB-Konzentration gesenkt, kann der Farbumschlag bei niedrigerem H₂O₂-Umsatz erreicht werden, wodurch auch sehr niedrige Konzentrationen des zu bestimmenden Antigens genau quantifiziert werden können. Die Untergrenze wird durch die Wahrnehmbarkeit des Farbstoffes mit dem bloßen Auge definiert und liegt bei 10 nmol/ml. Das Verfahren kann mit Vorteil bei einer Farbstoffkonzentration im Bereich von 10 bis 250 nmol/ml verwendet werden. Bevorzugt ist eine Verwendung bei einer Farbstoffkonzentration im Bereich von 10 bis 100 nmol/ml. Der Test ist also sehr variabel und kann je nach Anwendung auf hohe Empfindlichkeit oder großen Meßbereich ausgelegt werden.

Zur Durchführung bestehen prinzipiell zwei Möglichkeiten:
a) Als Indikatorenzym kann Peroxidase verwendet werden, als Farbsubstrat ein Redoxindikator. Bevorzugt wird als Farbstoff TMB eingesetzt. Die Konzentration des TMB wird dabei so gewählt, daß sie der oberen Grenze des Meßbereiches entspricht Dadurch ändert sich mit zunehmender Enzymkonzentration und entsprechend zunehmendem H₂O₂-Umsatz nicht nur die Konzentration eines farbigen Produktes, sondern auch das Verhältnis zweier unterschiedlich gefärbter Produkte und dadurch die spektrale Zusammensetzung der zu betrachtenden Farbe.
b) In der zweiten Version können als Indikatorenzym eine Oxidase in einem gekoppelten System mit Peroxidase verwendet werden. Bevorzugt werden Glucose-Oxidase markierte Antikörper verwendet. Glucose-Oxidase setzt Glucose, die in optimaler Konzentration eingesetzt werden kann, mit Sauerstoff zu Gluconsäure um, wobei H₂O₂ als Nebenprodukt entsteht, dessen Konzentration mit TMB und Peroxidase bestimmt wird. Dabei kann die Peroxidase in so hohen Konzentrationen eingesetzt werden, daß das H₂O₂ innerhalb weniger Minuten quantitativ umgesetzt wird. Durch den entstehenden Farbton kann also die gebildete H₂O₂-Konzentration und damit die Menge der gebundenen Glucose-Oxidase und die Konzentration des nachzuweisenden Antigens bestimmt werden.

Der Test läßt sich prinzipiell zum Nachweis von Antikörpern und allen Substanzen verwenden, für die Antiseren verfügbar sind; beispielsweise zum Nachweis von Bakterien und Viren in der Biotechnologie, Lebensmittelanalytik, Hygiene, Medizin und Umwelt, aber auch zum Nachweis von verschiedenen Giften, Hormonen, Pharmaka etc. Sinnvolle Anwendung findet er überall, wo es gilt, schnell und ohne Laborausstattung quantitative Bestimmungen durchzuführen. Die Erfindung wird anhand einiger Beispiele näher erläutert.

### 1. Biotechnologie

Die Probe aus dem Reaktor wird mit zuvor immobilisierten Antikörpern gegen den spezifischen Stamm inkubiert. Die Bakterien dieses Stammes werden gebunden, die anderen werden ebenso wie alle übrigen Substanzen aus der Probe ausgewaschen. Die gebundenen Bakterien werden mit Glucose-Oxidase konjugierten Antikörpern markiert. Die Glucose-Oxidase wird durch den Substratpuffer nachgewiesen, der Glucose, Peroxidase und TMB enthält. Nach festgelegten Reaktionszeiten werden Proben entnommen und nach wenigen Minuten, nach Abschluß der Peroxidase-Reaktion, mit Referenzfarben verglichen und damit die Konzentration des spezifischen Bakterienstammes bestimmt.

### 2. Cholesterinspiegel

Die Arteriosklerose, mit ihren gefährlichen Folgen wie Herzinfarkt und Schlaganfall, die häufigste Todesursache in der westlichen Welt, ist auf Einlagerungen von Cholesterin in Gefäßwänden zurückzuführen, das aus LDL (low density lipoprotein)-Partikeln im Blut stammt. Der LDL-Spiegel ist direkt mit dem Risiko einer Arteriosklerose korreliert. Die Risikoabschätzung ist also durch Bestimmung des LDL-Spiegels möglich. Die Bestimmung kann durchgeführt werden mit Hilfe enzymmarkierter Antikörper gegen Cholesterin oder Apoprotein B-100, dem Protein in LDL-Partikeln, das durch LDL-Rezeptoren in der Membran tierischer Zellen spezifisch erkannt wird.

### 3. Nachweis von Pharmaka Beispiel: Herzglycoside

Herzwirksame Glycoside werden zur Therapie der Herzinsuffizienz verwendet, um Schlagvolumen und Minutenvolumen des Herzens zu steigern. Die Dosierung der Herzglycoside muß individuell bestimmt werden und sehr genau erfolgen. Man unterscheidet die Sättigungsdosis, die zum Erreichen der gewünschten Wirkung verabreicht werden muß, und die Erhaltungsdosis, die zur Erhaltung des Vollwirkspiegels nötig ist. Die Erhaltungsdosis hängt von der Resorptions- und Abklingquote des einzelnen Glycosides beim einzelnen Patienten ab. Es wird vermutet, daß ca. 20 % der mit Herzglycosiden behandelten Patienten an einer Überdosierung dieser Glycoside sterben.

Die Serumkonzentration und damit die Resorptions- und Abklingquote lassen sich durch diesen Test mit Hilfe GOD-markierter Antikörper gegen Herzglycoside schnell und exakt in jeder Arztpraxis bestimmen.

### 4. Nachweis von Hormonen Beispiel: Luteinisierendes Hormon (LH)

Zum Zeitpunkt der Ovulation steigt der LH-Spiegel im Serum der Frau steil an und bleibt ca. 24 Stunden bestehen, um wieder ebenso rasch abzufallen. Im Rahmen ärztlicher Hilfe bei unerfülltem Kinderwunsch kann die betroffene Frau mit einem immunenzymatischen Nachweis von LH im Urin den exakten Zeitpunkt des Eisprungs selbst bestimmen. Im Gegensatz zum Schwangerschaftstest durch immunologischen β-HCG-Nachweis muß dieser Test quantitativ sein, da der Zeitpunkt der LH-Konzentrationsmaximums dem Ovulationszeitpunkt entspricht.

### 5. Nachweis von Antikörpern Beispiel: Allergietest

Charakteristisch für Allergien ist die Bildung von IgE-Antikörpern, die spezifisch gegen das betreffende Allergen gerichtet sind. Ein einfacher immunenzymatischer Allergietest kann durchgeführt werden, indem die infrage kommenden Allergene jeweils an eine feste Oberfläche gebunden werden und mit einer Serumprobe des Patienten inkubiert werden. IgE-Antikörper, die an das Allergen gebunden wurden, lassen sich durch enzymmarkierte Anti-Human-IgE-Antikörper nachweisen. Eine starke Enzymkonzentration kennzeichnet das Allergen, gegen das der Patient sensibilisiert ist. Ein solcher immunenzymatischer Allergietest ist völlig risikofrei und für den Patienten wesentlich angenehmer als der herkömmliche Allergietest, bei dem durch subcutane Applikation der Allergene eine allergische Hautreaktion erzeugt wird.

## Patentansprüche

1. Verfahren zur immunenzymatischen Bestimmung von Antigenen mit einem ersten Antikörper oder von Antikörpern mit einem zweiten Antikörper und einem an den ersten bzw. zweiten Antikörper kovalent gebundenen Indikatorenzym mittels eines Farbstoffes, indem eine Farbreaktion mit dem Indikatorenzym durchgeführt wird,
dadurch gekennzeichnet,
daß die Farbstoffkonzentration nur in der Größenordnung des Substratumsatzes liegt, so daß in Abhängigkeit der Antigen- bzw. Antikörperkonzentration unterschiedlich gefärbte Reaktionsprodukte entstehen, und daß die quantitative Bestimmung des Antigens bzw. Antikörpers durch einen visuellen Vergleich der farbigen Reaktionsprodukte mit Referenzfarben mit dem menschlichen Auge erfolgt.

2. Verfahren nach Anspruch 1, dadurch gekennzeichnet, daß eine Farbstoffkonzentration im Bereich von 10 bis 250 nmol/ml verwendet wird.

3. Verfahren nach Anspruch 1 - 2, dadurch gekennzeichnet, daß bevorzugt eine Farbstoffkonzentration im Bereich von 10 bis 100 nmol/ml verwendet wird.

4. Verfahren nach Anspruch 1 - 3, dadurch gekennzeichnet, daß als Indikatorenzym Peroxidase verwendet wird.

5. Verfahren nach Anspruch 1 - 3, dadurch gekennzeichnet, daß als Indikatorenzym eine Oxidase in einem gekoppelten System mit Peroxidase verwendet wird.

6. Verfahren nach Anspruch 1 - 3, dadurch gekennzeichnet, daß als Indikatorenzym Glucose-Oxidase in einem gekoppelten System mit Peroxidase verwendet wird.

7. Verfahren nach Anspruch 1 - 6, dadurch gekennzeichnet, daß als Farbstoff ein Redoxindikator verwendet wird.

8. Verfahren nach Anspruch 7, dadurch gekennzeichnet, daß als Farbstoff 3,3',5,5'-Tetramethylbenzidin verwendet wird.

9. Verfahren nach einem der Ansprüche 1 bis 8, dadurch gekennzeichnet, daß ein Farbstoff verwendet wird, der unterschiedlich gefärbte Reaktionsprodukte zu bilden vermag, die Farbstoffkonzentration nur in der Größenordnung des Substratumsatzes gehalten wird, so daß in Abhängigkeit der Antigen- bzw. Antikörperkonzentration unterschiedlich gefärbte Reaktionsprodukte entstehen, und daß zur Variation des Meßbereiches die Farbstoffkonzentration so gewählt wird, daß der Farbumschlag zur Detektion ausnützbar ist, indem die quantitative Bestimmung des Antigens bzw. Antikörpers durch den visuellen Vergleich der farbigen Reaktionsprodukte mit Referenzfarben erfolgt.

## Claims

1. Process for the immunoenzymatic assay of antigens with a first antibody or antibodies with a second antibody and an indicator enzyme covalently bonded to the first or second antibody by means of a dye, in that a colour reaction is performed with the indicator enzyme, characterized in that the dye concentration is only of the same order of magnitude as the substrate turnover, so that as a function of the antigen or antibody concentration differently coloured reaction products are formed and that the quantitative assay of the antigen or antibody takes place by a visual comparison of the coloured reaction products with reference colours using the human eye.

2. Process according to claim 1, characterized in that a dye concentration in the range 10 to 250 nmole/ml is used.

3. Process according to claims 1 or 2, characterized in that preferably a dye concentration in the range 10 to 100 nmole/ml is used.

4. Process according to claims 1 to 3, characterized in that peroxidase is used as the indicator enzyme.

5. Process according to claims 1 to 3, characterized in that an oxidase in a coupled system with peroxidase is used as the indicator enzyme.

6. Process according to claims 1 to 3, characterized in that glucose oxidase in a coupled system with peroxidase is used as the indicator enzyme.

7. Process according to claims 1 to 6, characterized in that a redox indicator is used as the dye.

8. Process according to claim 7, characterized in that 3,3',5,5'-tetramethylbenzidine is used as the dye.

9. Process according to one of the claims 1 to 8, characterized in that a dye is used, which is able to form differently coloured reaction products, the dye concentration is kept only at the same order of magnitude as the substrate turnover, so that as a function of the antigen or antibody concentration differently coloured reaction products are formed and that for varying the measuring range the dye concentration is chosen in such a way that the colour change can be used for detection purposes, in that the quantitative assay of the antigen or antibody takes place by visual comparison of the coloured reaction products with reference colours.

## Revendications

1. Procédé de détermination immuno-enzymatique des antigènes avec un premier anticorps ou des anticorps avec un deuxième anticorps et un enzyme indicateur lié au premier ou au deuxième anticorps par covalence à l'aide d'une matière colorante, grâce à une réaction colorée avec l'enzyme indicateur. Ce procédé est caractérisé par le fait que la concentration de matière colorante se situe seulement dans l'ordre de grandeur de la conversion de substrat de telle sorte que les produits de réaction sont de coloration différente selon la concentration en antigènes ou en anticorps, et que la détermination quantitative des antigènes ou des anticorps résulte d'une comparaison visuelle à l'oeil nu des produits de réaction colorés avec les couleurs de référence.

2. Procédé, d'après la revendication 1, caractérisé par le fait que la concentration de matière colorante utilisée se situe entre 10 et 250 nmol/ml.

3. Procédé, d'après les revendications 1 et 2, caractérisé par le fait que la concentration de matière colorante utilisée se situe de préférence entre 10 et 100 nmol/ml.

4. Procédé, d'après les revendications 1 à 3, caractérisé par le fait que l'enzyme indicateur utilisé est la peroxydase.

5. Procédé, d'après les revendications 1 à 3, caractérisé par le fait que l'enzyme indicateur utilisé est une oxydase dans un système couplé à la peroxydase.

6. Procédé, d'après les revendications 1 à 3, caractérisé par le fait que l'enzyme indicateur utilisé est une glucose-oxydase dans un système couplé à la peroxydase.

7. Procédé, d'après les revendications 1 à 6, caractérisé par le fait que la matière colorante utilisée est un indicateur redox.

8. Procédé, d'après la revendication 7, caractérisé par le fait que la matière colorante utilisée est de la benzidine tétraméthyle 3,3',5,5'.

9. Procédé, d'après les revendications 1 à 8, caractérisé par le fait qu'une matière colorante utilisée est capable de créer des produits de réaction de différentes couleurs, la concentration de matière colorante se situe seulement dans l'ordre de grandeur de la conversion de substrat de telle sorte que les produits de réaction sont de coloration différente selon la concentration en antigènes ou en anticorps, et que pour la variation de la plage de mesure la concentration de matière colorante est choisie de telle sorte que le changement de couleur est utilisable pour la détection, c'est-à-dire la détermination quantitative des antigènes ou des anticorps par une comparaison visuelle à l'oeil nu des produits de réaction colorés avec les couleurs de référence.
